# EUROPEAN PATENT APPLICATION

(11) **EP 2 719 691 A1**
(43) Date of publication of application: **16.04.2014**
(21) Application number: 11866737.7
(22) Date of filing: 15.06.2011
(51) Int. Cl.: C07D 279/08, C07D 417/12, C07D 417/04, C07D 413/04, A61K 31/5415, A61P 31/06

(54) **BENZOTHIAZINETHIONE DERIVATIVES AND THEIR PREPARATIVE METHODS AND USES**

(30) Priority: 27.05.2011 CN 201110139840
(71) Applicant: Si Chuan University, Sichuan 610065 (CN)
(72) Inventor: YU, Luoting, Chengdu Sichuan 610065 (CN); WEI, Yuquan, Chengdu Sichuan 610065 (CN)
(74) Representative: McCarthy, Denis Alexis
(86) International application number: PCT/CN2011/075750
(87) International publication number: WO 2012/162912

(57) **Abstract**

The invention belongs to the medicine field, and particularly relates to benzothiazinethione derivatives and preparation methods and uses thereof. In the aspect of the present invention, novel benzothiazinethione derivatives of formula I are provided, the benzothiazinethione derivatives of the invention are new compounds obtained based on extensive screening. Experimental results show that the benzothiazinethione derivatives of formula I have obvious inhibitory effects on mycobacterium tuberculosis, with effects equivalent to or even better than that of isoniazide (MIC₉₀=0.8µM). The benzothiazinethione derivatives of formula I have anti-mycobacterium tuberculosis activities, and provide new choices for the development and application of antitubercular agents.

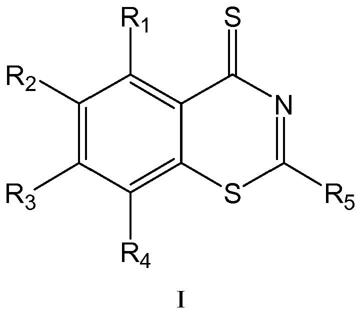

## Description

### Field of the Invention

The invention belongs to the medicine field, and particularly relates to benzothiazinethione derivatives and preparation methods and uses thereof.

### Description of the Related Art

Tuberculosis (TB) is one of diseases with the highest prevalence and mortality in history. In the twenty-first century, TB is still a main disease causing death in developing countries and a reactive disease in developed countries. Due to poverty and the prevalence of HIV/AIDS, and occurrence of multidrug-resistant tuberculosis (MDR-TB) and extensively drug-resistant tuberculosis (XDR-TB), the global death toll from TB increases continuously, and existing antituberculotics cannot satisfy the requirements for curing TB. At present, one third of the world's population (i.e. 2 billion persons) carries mycobacterium tuberculosis, pulmonary TB kills 3 million persons every year, and TB is sweeping across the world. As one of developing countries, China has about 4.5 million patients with active pulmonary TB, and the number of patients ranks second in the world. Traditional TB treatment cycle is long, bringing great pressure to the society and families, and restricting the sustainable development of economy in China to a certain extent. The success of research and development of new antituberculotics mainly depends on knowledge about the complex mechanisms of action on mycobacterium tuberculosis and human host cells, that is, selection of target and success in designing specificity of inhibitors or activators for the target. Vadim Makarov, et al. reported DprE₁ enzyme as target of anti-mycobacterium tuberculosis cell wall inhibitor in the journal *Science* for the first time. DprE₁ enzyme is a key enzyme for synthesizing araban which is an essential component of the mycobacteria cell wall. Compounds inhibit DprE₁, blocking synthesis of DPA which is an important precursor for synthesis of araban, further blocking synthesis of the araban, disabling synthesis of mycobacterium tuberculosis cell wall, then bacterial cells dissolve, killing mycobacterium tuberculosis, thus DprE₁ can become a new drug target different from existing antituberculotic target.

Since DprE₁ as new antituberculotic target was proposed, the inventor of the invention has carried out design and synthesis research on targeted small molecular drugs for DprE₁ enzyme, designed and synthesized a series of solid compounds, found that some compounds have certain inhibitory activity for mycobacterium tuberculosis through in vitro cell screening, and obtained some new compounds by further structural optimization and synthesis. The new compounds show excellent inhibitory activity and good results in in vivo tests.

### Summary of the Invention

The first technical problem to be solved by the invention is to provide a kind of new benzothiazinethione derivatives of structural formula I: 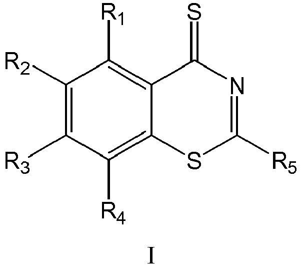 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃;
R₅ is 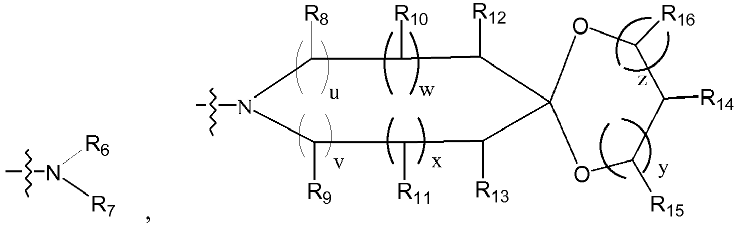 or 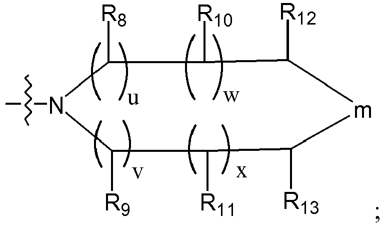 R₆ and R₇ are independently H, C1 - C8 alkyl with substituent, halogen substituted C1-C8 alkyl with substituent, phenyl with substituent or pyridyl with substituent; the substituent is H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl;
R₈ - R₁₆ are independently H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, OCF₃, OH, CF₃ or phenyl;
m is N, O or S; and u=0-1, v=0-1, w=0-1, x=0-1, y=0-1, z=0-1;
preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, NH₂, CN or CF₃;
R₅ is 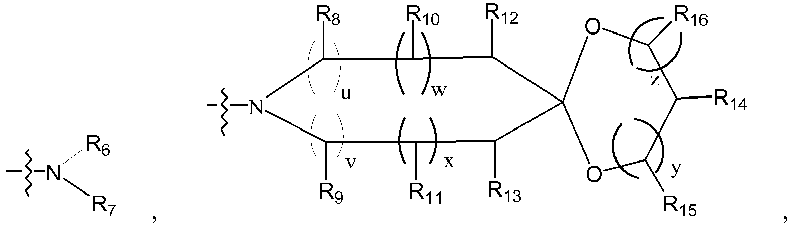 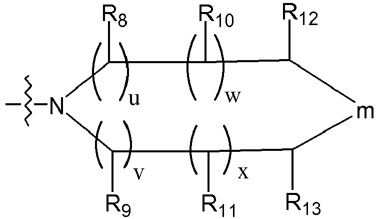 or 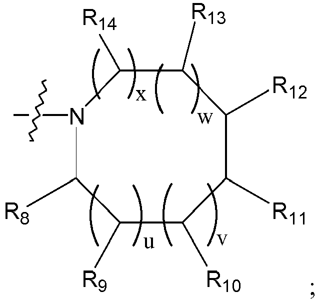 R₆ and R₇ are independently H, C1 - C8 alkyl, halogen substituted C1 - C8 alkyl, phenyl with substituent, or pyridyl with substituent; and the substituent is H, C1 - C8 alkyl, halogen substituted C1 - C8 alkyl, F, Cl, Br, CF₃, OCF₃, NO₂, NH₂ or CN;
R₈ - R₁₆ are independently H, F, Cl, Br, C1 - C8 alkyl or halogen substituted C1 - C8 alkyl;
m is N, O or S; and u=0-1, v=0-1, w=0-1, x=0-1, y=0-1, z=0-1.
more preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, NH₂, CN or CF₃;
R₅ is 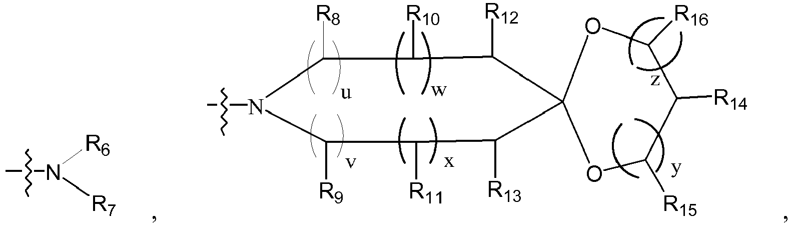 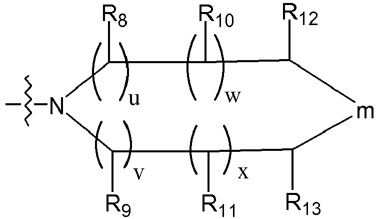 or 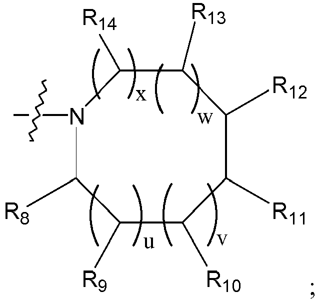 R₆ and R₇ are independently H, C1 - C8 alkyl, halogen substituted C1 - C8 alkyl, phenyl with substituent, or pyridyl with substituent; the substituent is H, C1 - C8 alkyl, halogen substituted C1 - C8 alkyl, F, Cl, Br, CF₃, OCF₃, NO₂, NH₂ or CN;
R₈ - R₁₆ are independently H, C1 - C8 alkyl or halogen substituted C1 - C8 alkyl;
m is O; and u=0-1, v=0-1, w=0-1, x=0-1, y=0-1, z=0-1.

More preferably, R₁ - R₄ are independently H, F, Cl, Br, C1 - C8 alkyl, CF₃ or NO₂;
R₅ is 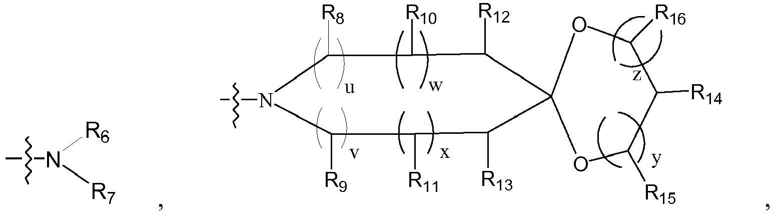 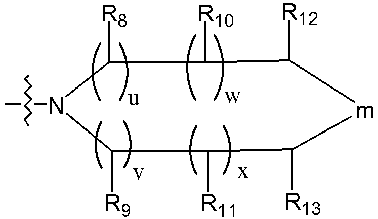 or 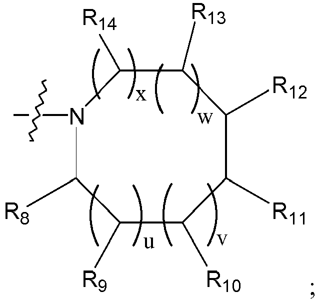 R₆ and R₇ are independently H, C1 - C8 alkyl, phenyl with substituent or pyridyl with substituent; the substituent is H, C1 - C8 alkyl, NO₂, F, Cl, Br or CF₃;
R₈ - R₁₆ are H ; and m is O, u=v=z=0, w=x=y=1.

Most preferably, the benzothiazinethione derivatives are:
2-((5-bromopyridine-2-amino)-6,7,8-trifluoro-4H-benzo[e][1,3]thiazine-4-thione,
6,8-dinitro-2-(4-(trifluoromethyl)anilino)-4H-benzo[e][1,3]thiazine-4-thione,
2-((ethylamino)-6,8-dinitro-4H-benzo[e][1,3]thiazine-4-thione,
2-((methylamino)-6,8-dinitro-4H-benzo[e][1,3]thiazine-4-thione,
6,8-dinitro-2-(piperidine-1-alkyl)-4H-benzo[e][1,3]thiazine-4-thione,
8-nitro-2-(1,4-dio-8-aza[4.5]dec-8-yl)-6-(trifluoromethyl)-4H-benzo[e][1,3]thiazine-4-thio ne,
2-morpholinyl-6,8-dinitro-4H-benzo[e][1,3]thiazine-4-thione,
or 2-morpholinyl-8-nitro-6-(trifluoromethyl)-4H-benzo[e][1,3]thiazine-4-thione.

Further, the benzothiazinethione derivative of structural formula II: 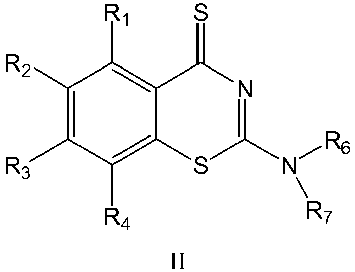 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃;
R₆ and R₇ are independently H, C1 - C8 alkyl with substituent, halogen substituted C1-C8 alkyl with substituent, phenyl with substituent or pyridyl with substituent; and the substituent is H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl;
or R₆ and R₇ are bridged as 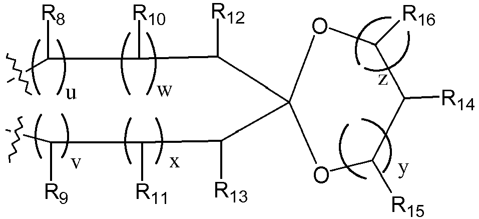 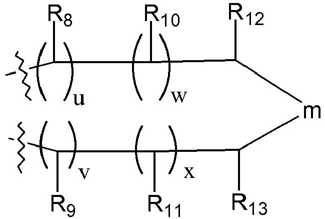 or 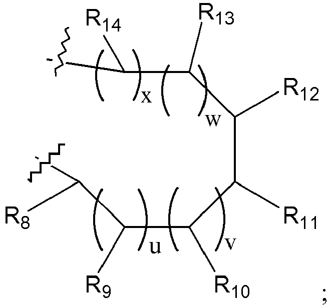 R₈ - R₁₆ are independently H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, OCF₃, OH, CF₃ or phenyl;
m is N, O or S; and u=0-1, v=0-1, w=0-1, x=0-1, y=0-1, z=0-1.

More preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, NH₂, CN or CF₃;
R₆ and R₇ are independently H, C1 - C8 alkyl, halogen substituted C1 - C8 alkyl, phenyl with substituent, or pyridyl with substituent; and the substituent is H, C1 - C8 alkyl, halogen substituted C1 - C8 alkyl, F, Cl, Br, CF₃, OCF₃, NO₂, NH₂ or CN;
or R₆ and R₇ are bridged as 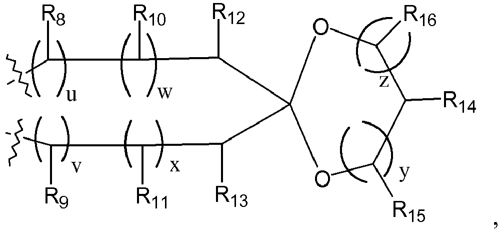 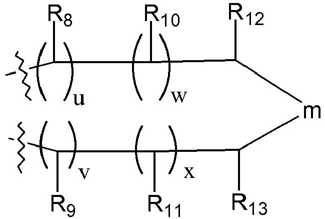 or 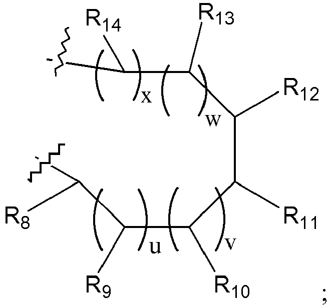 R₈ - R₁₆ are independently H, F, Cl, Br, C1 - C8 alkyl or halogen substituted C1 - C8 alkyl;
m is N, O or S; and u=0-1, v=0-1, w=0-1, x=0-1, y=0-1, z=0-1.

More preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, NH₂, CN or CF₃;
R₆ and R₇ are independently H, C1 - C8 alkyl, halogen substituted C1 - C8 alkyl, phenyl with substituent, or pyridyl with substituent; and the substituent is H, C1 - C8 alkyl, halogen substituted C1 - C8 alkyl, F, Cl, Br, CF₃, OCF₃, NO₂, NH₂ or CN; or R₆ and R₇ are bridged as 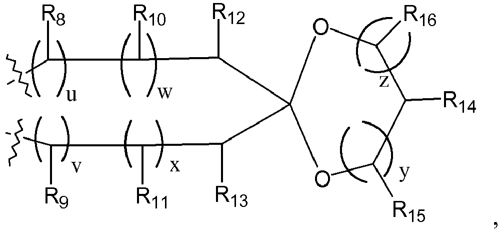 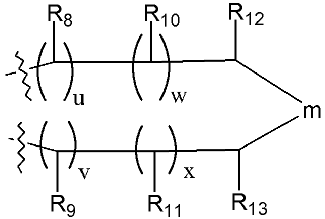 or 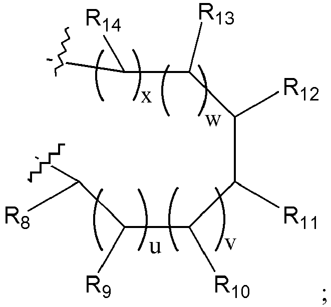 R₈ - R₁₆ are independently H, C1 - C8 alkyl or halogen substituted C1 - C8 alkyl; m is O; and u=0-1, v=0-1, w=0-1, x=0-1, y=0-1, z=0-1.

Most preferably, R₁ - R₄ are independently H, F, Cl, Br, C1 - C8 alkyl, CF₃ or NO₂;
R₆ and R₇ are independently H, C1 - C8 alkyl, phenyl with substituent or pyridyl with substituent; and the substituent is H, C1 - C8 alkyl, NO₂, F, Cl, Br or CF₃;
R₈ - R₁₆ are H; and m is O, u=v=z=0, w=x=y=1.

Further, the benzothiazinethione derivative of structural formula III: 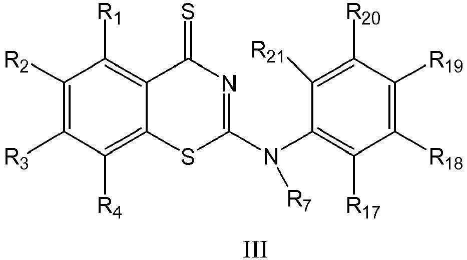 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃;
R₇ is independently H, C1 - C8 alkyl with substituent, halogen substituted C1 - C8 alkyl with substituent, phenyl with substituent or pyridyl with substituent; and the substituent is H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl; and
R₁₇ - R₂₁ are independently H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl.

Preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, OCF₃, NO₂, CN or CF₃;
R₇ is independently H, C1 - C8 alkyl, halogen substituted C1 - C8 alkyl, phenyl with substituent, or pyridyl with substituent; the substituent is H, F, Cl, Br, CF₃, NO₂, C1 - C8 alkyl or halogen substituted C1 - C8 alkyl; and
R₁₇ - R₂₁ are independently H, F, Cl, Br, CF₃, NO₂, C1 - C8 alkyl or halogen substituted C1 - C8 alkyl.

More preferably, R₁ - R₄ are independently H, F, Cl, Br, C1 - C8 alkyl, halogen substituted C1 - C8 alkyl, NO₂ or CF₃;
R₇ is independently H, C1 - C8 alkyl, halogen substituted C1 - C8 alkyl, phenyl with substituent, or pyridyl with substituent; the substituent is H, F, Cl, Br, CF₃, NO₂, C1 - C8 alkyl or halogen substituted C1 - C8 alkyl; and
R₁₇ - R₂₁ are independently H, F, Cl, Br, CF₃, NO₂, C1 - C8 alkyl or halogen substituted C1 - C8 alkyl.

Most preferably, R₁ - R₄ are independently H, C1 - C8 alkyl or NO₂;
R₇ is independently H or C1 - C8 alkyl; and
R₁₇ - R₂₁ are independently H, CF₃ or C1 - C8 alkyl.

Further, the benzothiazinethione derivative of structural formula IV: 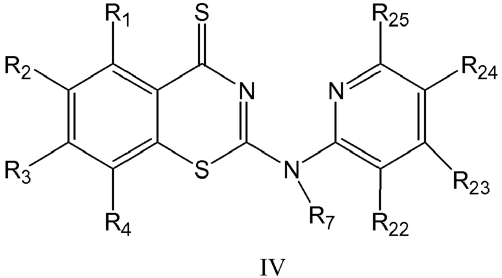 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃;
R₇ is independently H, C1 - C8 alkyl with substituent, halogen substituted C1 - C8 alkyl with substituent, phenyl with substituent or pyridyl with substituent; the substituent is H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl; and
R₂₂ - R₂₅ are independently H, C1 - C8 alkyl, C1 - C8 alkoxyl, C1 - C8 alkyl substituted sulfamoyl, halogen substituted C1 - C8 alkyl, C1 - C8 alkyl substituted carbonyl, C1 - C8 alkyl substituted aminoacyl, C1 - C8 alkyl substituted acylamino, halogen, NO₂, OH, OCF₃, CF₃ or phenyl.

Preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, OCF₃, NO₂, CN or CF₃;
R₇ is independently H, C1 - C8 alkyl or halogen substituted C1 - C8 alkyl; and R₂₂ - R₂₅ are independently H, F, Cl, Br, CF₃, NO₂, C1 - C8 alkyl or halogen substituted C1 - C8 alkyl.

Most preferably, R₁ - R₄ are independently H, F, Cl, Br or C1 - C8 alkyl;
R₇ is independently H or C1 - C8 alkyl; and
R₂₂ - R₂₅ are independently H, F, Cl, Br or C1 - C8 alkyl.

Further, the benzothiazinethione derivative of structural formula V: 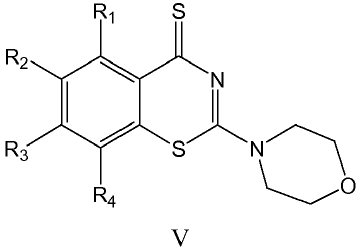 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃.

Preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, OCF₃ or CF₃.

Preferably, R₁ - R₄ are independently H, C1 - C8 alkyl, CF₃ or NO₂.

Further, the benzothiazinethione derivative of structural formula VI: 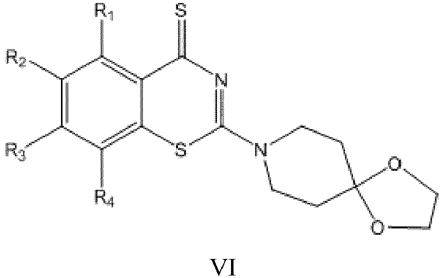 wherein, R₁ - R₄ are independently H, halogen, C1 - C8 alkyl, C1 - C8 alkoxyl, halogen substituted C1 - C8 alkyl, halogen substituted C1 - C8 alkoxyl, C1 - C8 alkyl substituted amino, C1 - C8 alkyl substituted carbonyl, C1 - C8 alkyl substituted aminoacyl, C1 - C8 alkyl substituted acylamino, C1 - C8 alkyl substituted sulfamoyl, NO₂, NH₂, OCF₃, CN, OH, CHO or CF₃.

Preferably, R₁ - R₄ are independently H, F, Cl, Br, C1 - C8 alkyl, C1 - C8 alkoxyl, halogen substituted C1 - C8 alkyl, halogen substituted C1 - C8 alkoxyl, NO₂, OCF₃ or CF₃.

Most preferably, R₁ - R₄ are independently H, C1 - C8 alkyl, CF₃ or NO₂.

The second technical problem to be solved by the invention is to provide a method for synthesizing the compound shown in the formula I, as follows:

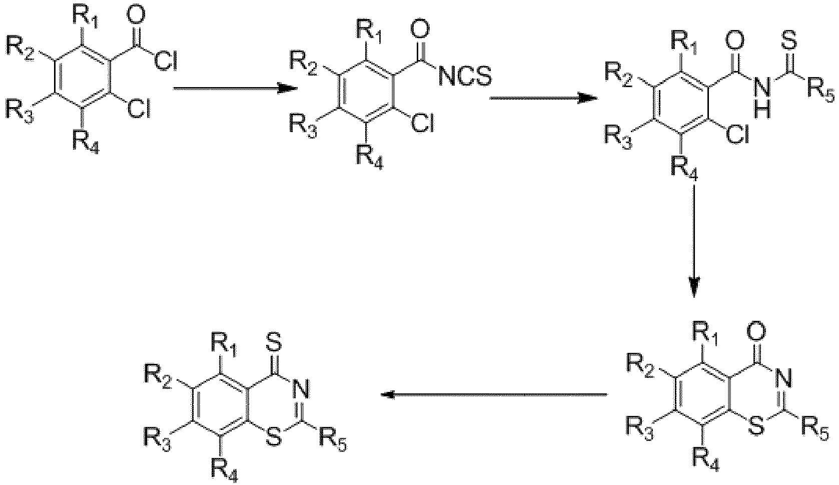

R₁ - R₄ substituted benzoyl chloride reacts with ammonium thiocyanate in the presence of a catalyst to obtain R₁ - R₄ substituted benzoyl isothiocyanate, then the R₁ - R₄ substituted benzoyl isothiocyanate obtained reacts with R₅H by cyclization to obtain R₁ - R₄ substituted benzothiazinone, and finally the R₁ - R₄ substituted benzothiazinone obtained reacts with Lawesson's reagent to obtain R₁ - R₄ substituted benzothiazinethione.

The catalyst is 18-crown-6 or PEG. PEG is preferably PEG-400 or PEG-300.

The solvent used in reaction with ammonium thiocyanate is dichloromethane or toluene.

The solvent used in reaction with Lawesson's reagent is toluene.

The reaction temperature in each reaction step is normal temperature.

The third technical problem to be solved in the invention is to provide use of the benzothiazinethione derivative shown in formula I in antituberculotics.

Experimental results show that the benzothiazinethione derivative of formula I has obvious inhibitory effects on mycobacterium tuberculosis, with effects equivalent to or even better than those of isoniazide (IC₉₀=0.8[µM).

The fourth technical problem to be solved by the invention is to provide a pharmaceutical composition prepared from the benzothiazinethione derivative of formula I and pharmaceutically acceptable auxiliary components. The pharmaceutical composition can be used for preparing antituberculotics.

The beneficial effects of the invention are as follows: the benzothiazinethione derivatives of the invention are new compounds obtained based on extensive screening, have anti-mycobacterium tuberculosis activities, and provide new choices for development and application of antituberculotics.

### Brief Description of the Drawings

Figure 1 is a statistical chart of colony count results after treatment for 28 days.
Figure 2 is a tectological chart of pathological examination.

### Description of the Preferred Embodiments

The invention will be further described in combination with examples. The examples are illustrative only instead of limitation to the invention in any way.

### Example 1: Preparation of compound I a:

### 2-(5-bromopyridine-2-amino)-6,7,8-trifluoro-4H-benzo[e][1,3]thiazine-4-thione

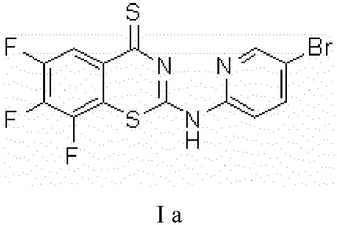

The 2,3,4,5-tetrafluorobenzoyl chloride (3g, 14.12mmol) was dissolved in dichloromethane (20ml), then ammonium thiocyanate (2.14g, 28.24mmol) was slowly added dropwise, and PEG-400 (0.2g) was added dropwise to obtain a solution, the solution was subject to reaction at normal temperature for 2h, then precipitate was filtered to obtain a filtrate, and the filtrate was slowly added dropwise to dichloromethane solution of 2-amino-5-bromo-pyridine (2.44g, 14.12mmol) for reaction at normal temperature for 3h. Water and dichloromethane were added to the reaction solution at room temperature to obtain a yellow intermediate by collecting and drying the organic layer in a rotary way, then the yellow intermediate was placed in a dry flask, and Lawesson's reagent (5.72g, 14.12mmol) and toluene (80ml) were added for refluxing for 40 minutes. The reaction solution was filtered after cooling, and the filtrate was subject to column chromatography to obtain 3.15g dark red solid (with yield of 55.1%).
¹H NMR: (DMSO-d6, 400MHz): 7.30(sbr, 1H), 8.12(dd, J=8.4, 2.0Hz, 1H), 8.51(t, J=8.4Hz, 1H), 8.64(s,1H)
MS-ESI(m/s):401.9(M-1), 403.9(M+1)

### Example 2: Preparation of compound I b:

### 6,8-dinitro-2-(4-(trifluoromethyl)anilino)-4H-benzo[e][1,3]thiazine-4-thione

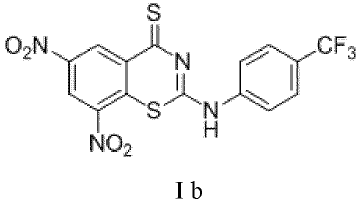

The 2-chloro-3,5-dinitrobenzoyl chloride (3g, 11.32mmol) was dissolved in toluene (20ml), then ammonium thiocyanate (1.71g, 22.64mmol) was slowly added dropwise, and 18-crown-6 (0.2g) was added to obtain a solution, the solution was subject to reaction at normal temperature for 2h, then precipitate was filtered to obtain a filtrate, and the filtrate was slowly added dropwise to toluene solution of p-trifluoromethylaniline (1.82g, 11.32mmol) for reaction at normal temperature for 2h. Water (20ml) was added to the reaction solution at room temperature, then the reaction solution was stirred for 30 minutes and extracted with ethyl acetate to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 3.20g red solid (with yield of 66.7%).
¹H NMR: (DMSO-d6, 400MHz): 7.20(s, 1H), 7.81(d, J=8.0Hz, 2H), 8.03(s, 1H), 9.17(s,1H), 11.60(s, 1H), 12.83(s, 1H),
MS-ESI(m/s):429.0(M+1)

### Example 3: Preparation of compound I c:

### 2-(ethylamino)-6,8-dinitro-4H-benzo[e][1,3]thiazine-4-thione

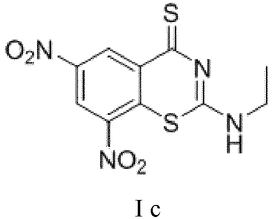

The 2-chloro-3,5-dinitrobenzoyl chloride (3g, 11.32mmol) was dissolved in dichloromethane (20ml), then ammonium thiocyanate (1.71g, 22.64mmol) was slowly added dropwise, and peg-400 (0.2g) was added to obtain a solution, the solution was subject to reaction at normal temperature for 2h, then precipitate was filtered to obtain a filtrate, and the filtrate was slowly added dropwise to ethylamine solution (0.51g, 11.32mmol) for reaction at normal temperature for 2h. Water (20ml) was added to the reaction solution at room temperature, then the reaction solution was stirred for 30 minutes and extracted with dichloromethane to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 1.04g red solid (with yield of 28.7%).
¹H NMR: (DMSO-d6, 400MHz): 1.23(t, J=7.2Hz, 3H), 3.60(m, 2H), 9.07(s, 1H), 9.71(s, 1H), 10.01(s, 1H)
MS-ESI(m/s):311.0(M-1), 313.0(M+1)

### Example 4: Preparation of compound I d:

### 2-(methylamino)-6,8-dinitro-4H-benzo[e][1,3]thiazine-4-thione

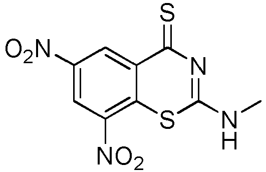

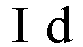

The 2-chloro-3,5-dinitrobenzoyl chloride (3g, 11.32mmol) was dissolved in dichloromethane (20ml), then ammonium thiocyanate (1.71g, 22.64mmol) was slowly added dropwise, and peg-300 (0.2g) was added to obtain a solution, the solution was subject to reaction at normal temperature for 2h, then precipitate was filtered to obtain a filtrate, and the filtrate was slowly added dropwise to methylamine solution (0.35g, 11.32mmol) for reaction at normal temperature for 2h. Water (20ml) was added to the reaction solution at room temperature, then the reaction solution was stirred for 30 minutes and extracted with dichloromethane to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 0.85g red solid (with yield of 25.8%).
¹H NMR: (DMSO-d6, 400MHz): 3.58(s, 1H), 8..72(s, 1H), 8.91(s, 1H), 10.43(s, 1H)
MS-ESI(m/s):297.0(M-1)

### Example 5: Preparation of compound I e:

### 6,8-dinitro-2-(piperidine-1-alkyl)-4H-benzo[e][1,3]thiazine-4-thione

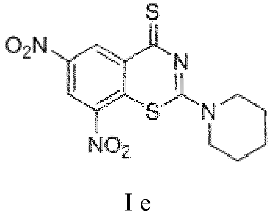

The 2-chloro-3,5-dinitrobenzoyl chloride (3g, 11.32mmol) was dissolved in dichloromethane (20ml), then ammonium thiocyanate (1.71g, 22.64mmol) was slowly added dropwise, and peg-400 (0.2g) was added to obtain a solution, the solution was subject to reaction at normal temperature for 2h, then precipitate was filtered to obtain a filtrate, and the filtrate was slowly added dropwise to hexahydropyridine (0.96g, 11.32mmol) for reaction at normal temperature for 2h. Water (20ml) was added to the reaction solution at room temperature, then the reaction solution was stirred for 30 minutes and extracted with dichloromethane to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 1.62g red solid (with yield of 40.5%).
¹H NMR: (DMSO-d6, 400MHz): 1.71(s, 6H), 3.85(s, 2H), 4.09(s, 2H), 9.07(s, 1H), 9.71(s, 1H)
MS-ESI(m/s): 353.0(M+1)

### Example 6: Preparation of compound I f:

### 8-nitro-2-(1,4-dio-8-aza[4.5]dec-8-yl)-6-(trifluoromethyl)-4H-benzo[e][1,3]thiazine-4-thio ne

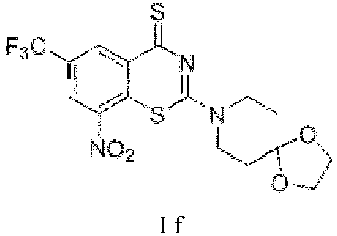

The 2-chloro-3-nitro-5-trifluoromethylbenzoyl chloride (3g, 10.41mmol) was dissolved in toluene (20ml), then ammonium thiocyanate (1.57g, 22.64mmol) was slowly added dropwise, and 18-crown-6 (0.2g) was added to obtain a solution, the solution was subject to reaction at normal temperature for 2h, then precipitate was filtered to obtain a filtrate, and the filtrate was slowly added dropwise to toluene solution of 4-piperidone ethylene ketal (1.49g, 10.41mmol) for reaction at normal temperature for 2h. Water (20ml) was added to the reaction solution at room temperature, then the reaction solution was stirred for 30 minutes and filtered to obtain a filter cake, then the filter cake was placed in a dry flask, and Lawesson's reagent (4.22g, 10.41mmol) and toluene (70ml) were added for refluxing for 30 minutes. The reaction solution was filtered after cooling, then the filtrate was condensed to obtain a crude product, and the crude product was subject to column chromatography to obtain 2.11g red solid (with yield of 46.9%).
¹H NMR: (DMSO-d6, 400MHz): 1.83(s, 4H), 3.90(s, 4H), 4.05(d, J=7.2, 4H), 8.81(s, 1H), 9.27(s, 1H)
MS-ESI(m/s): 434.1(M+1)

### Example 7: Preparation of compound I g:

### 2-morpholinyl-6,8-dinitro-4H-benzo[e][1,3]thiazine-4-thione

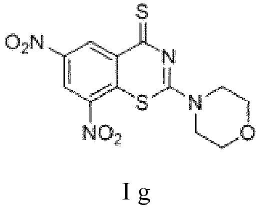

The 2-chloro-3,5-dinitrobenzoyl chloride (3g, 11.32mmol) was dissolved in toluene (20ml), then ammonium thiocyanate (1.71g, 22.64mmol) was slowly added dropwise, and 18-crown-6 (0.2g) was added to obtain a solution, the solution was subject to reaction at normal temperature for 2h, then precipitate was filtered to obtain a filtrate, and the filtrate was slowly added dropwise to toluene solution of morpholine (0.98g, 11.32mmol) for reaction at normal temperature for 2h. Water (20ml) was added to the reaction solution at room temperature, then the reaction solution was stirred for 30 minutes and extracted with ethyl acetate to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 1.41g red solid (with yield of 35.3%).
¹H NMR: (DMSO-d6, 400MHz): 3.77(s, 4H), 3.88(s, 2H), 4.03(s, 2H), 9.09(s, 1H), 9.70(s, 1H)
MS-ESI(m/s): 355.0(M+1)

### Example 8: Preparation of compound I h:

### 2-morpholinyl-8-nitro-6-(trifluoromethyl)-4H-benzo [e] [1,3]thiazine-4-thione

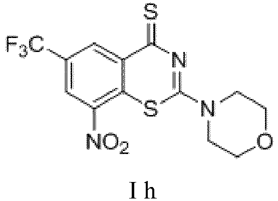

The 2-chloro-3-nitro-5-trifluoromethylbenzoyl chloride (3g, 10.41mmol) was dissolved in toluene (20ml), then ammonium thiocyanate (1.57g, 22.64mmol) was slowly added dropwise, and 18-crown-6 (0.2g) was added to obtain a solution, the solution was subject to reaction at normal temperature for 2h, then precipitate was filtered to obtain a filtrate, and the filtrate was slowly added dropwise to toluene solution of morpholine (0.91g, 10.41mmol) for reaction at normal temperature for 2h. Water (20ml) was added to the reaction solution at room temperature, then the reaction solution was stirred for 30 minutes and extracted with ethyl acetate to obtain a yellow intermediate by collecting and drying the organic layer in a rotary way, then the yellow intermediate was placed in a dry flask, and Lawesson's reagent (4.59g, 11.32mmol) and toluene (70ml) were added for refluxing for 30 minutes. The reaction solution was filtered after cooling, then the filtrate was condensed to obtain a crude product, and the crude product was subject to column chromatography to obtain 1.52g red solid (with yield of 39.0%).
¹H NMR: (DMSO-d6, 400MHz): 3.73(s, 4H), 3.92(s, 4H), 8.80(s, 1H), 8.86(s, 1H)
MS-ESI(m/s):375.9(M-1), 378.0(M+1)

### Pharmacodynamic experiments

I. In vitro inhibition test of drug on mycobacterium tuberculosis (H37Ra)
1. Preparation of inoculum for experiment: 5 *-* 10 H37Ra bacterial colonies and 1ml sterile saline solution prepared in advance were inoculated by a BBL pump to a kit for later use after growing for 2 - 3 weeks (as mycobacterium tuberculosis grows slowly); then approximate 10⁸CFU/ml mycobacterium tuberculosis suspension was obtained after ultrasonic treatment and volution, and specific test concentration can be obtained by dilution. The suspension was diluted by 200X by the following method: 0.2ml suspension containing H37Ra was added to 40ml sterile 7H9 broth containing 2% glycerinum and ADC (purchased from BD) nutrition additive (~10⁶ CFU/ml); then 100µl suspension (~5 x 10⁴ cells) was inoculated to microplate wells under test, and 1µl DMSO dissolved with the compound to be tested at certain concentration was added.
Compound dilution, inoculation, MIC test:
Isoniazide was selected for positive control, then the compounds to be tested and DMSO for positive control were prepared to 10mM solution, and successively diluted into 100µM, 50µM, 25µM, 12.5µM, 6.3µM, 3.1µM, 1.6µM, 0.8µM, 0.4µM, 0.2µM and 0.1µM for later use.
Then 1µl compound-DMSO solution diluted to different concentration was added to a 96-well plate, and 100µl diluted mycobacterium tuberculosis suspension was added, and evenly mixed manually by a dispensing gun.
The inoculated 96-well plate was incubated in a 5%CO₂ incubator at 37□ for 9 days.
After 9 days, 30µl 0.01% 7-hydroxyl-3H-phenoxazine-3-one-10-oxide was added to each well, then background fluorescence of each well was measured at 492nm, corresponding data were recorded, and the 96-well plate was replaced into the incubator for incubation for 24h.
The fluorescence of each well was measured at 492nm and corresponding data were recorded again after 24h.

2. Experimental results
See Table 1 for inhibitory effects of compounds Ia to Ih on mycobacterium tuberculosis (H37Ra):

**Table 1**

| Sample | Ia | Ib | Ic | Id | Ie | If | Ig | Ih | Isoniazide |
|---|---|---|---|---|---|---|---|---|---|
| MIC₉₀(µM) | 25 | 12.5 | 6.3 | 3.1 | 6.3 | 0.1 | 0.8 | 0.8 | 0.8 |

Results show that: Compounds Ia to Ih have obvious inhibitory effects, among which IC₉₀ of compounds If, Ig and Ih is 0.1µM, 0.8µM and 0.8µM respectively, with effects equivalent to or even better than those of isoniazide (IC₉₀=0.8[µM).
II. In vitro inhibition test of drug on mycobacterium tuberculosis (H37Rv)
1. Materials
1) Strain: Standard mycobacterium tuberculosis strain H37Rv was collected from American Type Culture Collection (ATCC 27294).
2) Liquid medium: Middlebrook 7H9 dehydrated medium and nutrition additive (OADC) purchased from BD.
3) Test drugs: Ia to Ih

2. Experimental method
1) Preparation of test strain
   Test strain was transferred to the liquid medium, activated and cultured at 37□ for 2 weeks, a small amount of the culture medium was absorbed and placed in 4ml liquid medium, then 10 - 20 sterile glass beads with diameter of 2 - 3mm were added, allowing oscillation for 20 - 30s, and static precipitation for 10 - 20min, then supernatant was absorbed, and the liquid medium was used for adjusting the turbidity to 1 MacConkey equivalent to 1 _{×} 10⁷CFU/ml for later use.
2) Preparation of test drugs
   Drugs were dissolved with proper amount of DMSO to 1mg/ml, filtered by a 0.22µm filter, and diluted with the liquid medium to desired test concentration (2× final concentration). Final concentrations of the test drugs were set as follows: 0.03125µg/ml, 0.0625µg/ml, 0.125µg/ml, 0.25µg/ml, 0.5µg/ml, 1µg/ml, 2µg/ml, 4µg/ml, 8µg/ml, 16µg/ml, 32µg/ml and 64µg/ml, with 12 concentration gradients in total.
3) Operating steps:
   At the time of detection, 0.1ml drug at each of above concentrations was taken respectively, added to a 96-well microplate, and 0.1ml culture medium at concentration of 10⁴CFU/ml (diluted from 10⁷CFU/ml) was added to allow the drug concentration to be 2 times the final concentration set. The culture medium was cultured at 37□, no drug was added to the blank control group, and three parallel control groups were set for each drug. The minimum inhibitory concentration (MIC90 and MIC99) of each drug on mycobacterium tuberculosis H37Rv was observed. (MIC90 refers to the drug concentration in drug wells similar to the growth of 10% inoculum size in control wells, and is generally observed 5 - 7 days after inoculation. MIC99 refers to the drug concentration in drug wells similar to the growth of 1% inoculum size in control wells, and is generally observed 11 days after inoculation.)

3. Experimental results: See Table 2 for inhibitory effects of compounds Ia to Ih on mycobacterium tuberculosis (H37Rv):

**Table 2**

| Sample | Ia | Ib | Ic | Id | Ie | If | Ig | Ih | Isoniazide |
|---|---|---|---|---|---|---|---|---|---|
| MIC₉₀ (µg/ml) | 8 | 4 | 2 | 2 | 1 | 0.03125 | 0.0625 | 0.03125 | 0.0625 |
| MIC₉₉ (µg/ml) | 16 | 16 | 8 | 4 | 4 | 0.03125 | 0.125 | 0.03125 | 0.0625 |

Results show that compounds Ia to Ih have obvious inhibitory effects, among which the effects of If, Ig and Ih are equivalent to or even better than those of isoniazide.
III. Cytotoxicity test
1. Test method (MTT method)
The 293 cells are human renal epithelial cells transfecting adenovirus E1A genes, thus 293 cells can be used for cytotoxicity test. Complete culture medium was used for adjusting cell concentration to 3×104/ml, then inoculated to a 96-well plate at 200ul each well for overnight culture, on the next day, the compounds Ia to Ih (with final concentration of 500, 400, 300, 200, 100, 50, 25, 12.5, 6.25, 3.125, 1.5625, 0.78125µM/l respectively) at different doses were respectively used for cell treatment, and meanwhile, a blank medium control group and a solvent control group of the same volume were set, with DMSO concentration of 0.5% (0.5% DMSO had no influence on cell proliferation). Each group was provided with 4 wells, and cultured in 5% CO₂ at 37□. After culture for 48h, each well was supplemented by 20µl 5mg/ml MTT reagent for continual culture for 2h, then supernatant was discarded, and 150µl DMSO was added, allowing oscillation and even mixing for 15min, then a microplate reader (OD=570nm) was used for determining absorbance (A) value (A value was proportional to viable cell count), taking the average value. Cellular proliferation inhibition rate (%)=(solvent control group A570-test group A570)/ solvent control group A570×100%. The inhibitory effects of all compounds below on cell proliferation are expressed by cellular proliferation inhibition rate (%).
2. Experimental results
See Table 3 for 293 cytotoxicity test results of compounds Ia to Ih.

**Table 3**

| Sample | Ia | Ib | Ic | Id | Ie | If | Ig | Ih |
|---|---|---|---|---|---|---|---|---|
| IC₅₀(µM) | >500 | >500 | >500 | >500 | >500 | >500 | >500 | >500 |

The results show that compounds Ia to Ih at concentration of 500µM have no obvious cytotoxicity through determination by MTT method.
IV. Acute toxicity test of compound If
1. Experimental method, 1) If, intraperitoneal suspension, at dose of 2.5g/kg; 2) adjuvant: 1% sodium carboxymethylcellulose (CMC-Na), 1% tween-80; 3) 10 BABL/C male and female mice, 6 - 8 weeks old, and weight of 20±2g. The 10 mice were randomly divided into 2 groups, 5 mice for each group, and a solvent control group is set. Single intraperitoneal injection of 2.5g/kg was given to the □ drug group and □ solvent control group within 24h; physical signs, behavioral activities, glandular secretion, breath, stool, genitals, death and other poisoning manifestations of the mice were constantly observed every 4h after administration, and the mice were killed after observation for 24h, 5) blood was taken from eyeballs, and then the mice were killed, 800µl whole blood was taken, centrifuged at 1300r/min for 15min, and 200µl* supernatant serum was taken for blood biochemical detection, including the following evaluation indicators: aspartate aminotransferase (AST), alanine aminotransferase (ALT), creatine kinase (CK), alkaline phosphatase (ALP), blood urea nitrogen (BUN ), total protein (TP), albumin (Alb), blood glucose (GLU), total bilirubin (T-BIL), creatinine (Crea), total cholesterol (Chol) and triglyceride (TG), with 12 items in total.
2. Experimental results: Test results of acute toxicity test of compound If are as shown in Table 4, and the results show that compound If at 2.5g/kg has no obvious acute toxicity in intraperitoneal injection.

**Table 4**

| | Solvent control group | | | | | Experimental results of compound If | | | | |
|---|---|---|---|---|---|---|---|---|---|---|
| | 1 | 2 | 3 | 4 | 5 | 6 | 7 | 8 | 9 | 10 |
| Albumin | 30.7 | 31 | 31.5 | 33.9 | 31.9 | 34.0 | 33.1 | 33.4 | 32.2 | 31.5 |
| Alkaline phosphatase | 162 | 172 | 161 | 170 | 182 | 134 | 156 | 175 | 165 | 166 |
| Alanine aminotransferase | 13 | 12 | 13 | 9 | 11 | 16 | 12 | 11 | 16 | 10 |
| Aspartate aminotransferase | 120 | 129 | 146 | 145 | 157 | 148 | 149 | 156 | 169 | 145 |
| Blood urea nitrogen | 7.3 | 8.3 | 6.5 | 8.7 | 7.7 | 5.5 | 6.3 | 8.9 | 7.9 | 6.7 |
| Total cholesterol | 3.15 | 3.02 | 3.45 | 3.41 | 3.00 | 3.21 | 3.34 | 3.53 | 3.14 | 3.35 |
| Creatine kinase | 51 | 58 | 84 | 57 | 75 | 54 | 65 | 66 | 74 | 67 |
| Creatinine | -4 | -5 | -14 | -13 | -11 | -6 | -12 | -15 | -4 | -12 |
| Blood glucose | 5.4 | 4.4 | 6.8 | 4.6 | 3.2 | 4.3 | 4.9 | 4.5 | 5.1 | 6.3 |
| Triglyceride | 3.10 | 2.38 | 1.47 | 2.23 | 2.30 | 1.80 | 2.53 | 2.17 | 2.24 | 2.10 |
| Total protein | 63.3 | 65.8 | 69.4 | 74.2 | 78.5 | 65.6 | 66.7 | 73.8 | 65.7 | 70.1 |
| Total bilirubin | -0.4 | -0.5 | -0.7 | -0.5 | -0.7 | -0.5 | -1.2 | -0.6 | -0.4 | -0.6 |

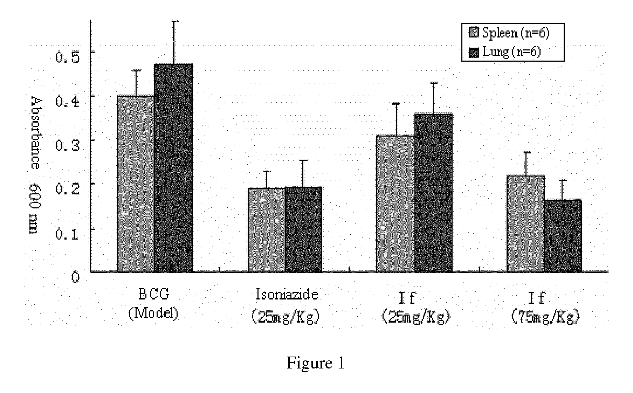

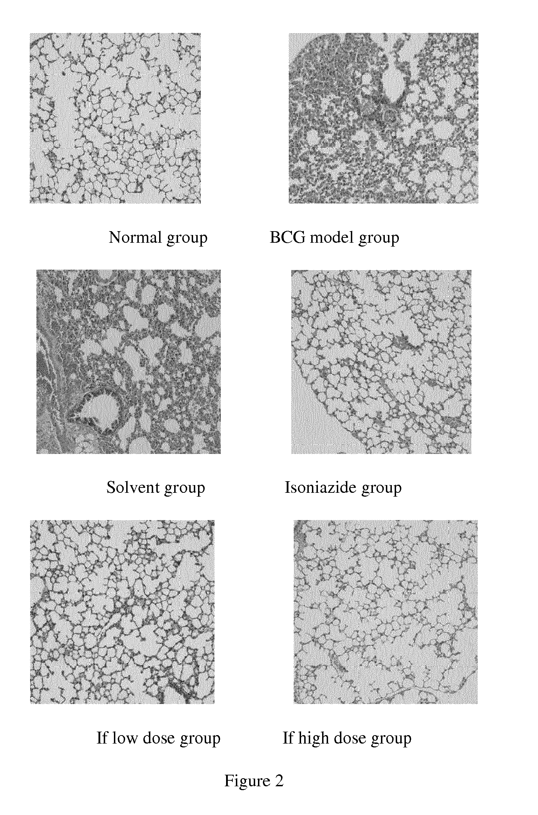

V. In vivo pharmacodynamic test of compound If against BCG
1 Laboratory animals
   BABL/C female mice, 6 - 8 weeks old, weight of 20±2g.
2 Strain
   Bacillus Calmette Guerin (BCG) injection provided by Chengdu Institute of Biological Products Co., Ltd.
3 Medium
   7H9 medium.
4 Preparation of culture medium
   BCG injection was inoculated to 7H9 culture medium, and cultured in a 37□ skin box shaker for 2 - 3 weeks for collecting bacteria, proper amount of bacteria was taken and diluted with saline containing 0.05% Tween-80, and the bacterial concentration was 2.5×10⁷CFU/ml when the absorbance value was tested (OD=600nm).
5 Procedures
   1) The 36 mice were randomly divided into 6 groups, 6 mice for normal control group, other mice were subject to tail intravenous injection of 0.1ml diluted culture medium, and then attack bacteria of each mouse is 1×10⁵CFU;
   2) Inoculated mice were grouped as a BCG model group, a solvent control group, an isoniazide (positive) control group, an If low dose group, and an If high dose group, with 6 mice for each group, and 5 groups in total;
   3) The solvent was 0.5% sodium carboxymethylcellulose (CMC-Na), 0.5% tween-80;
   4) Solvent control group: intraperitoneal injection of 0.1ml once every day;
      Isoniazide (positive) control group: intraperitoneal injection of 25mg/kg/10ml, 0.1ml once every day;
      If low dose group: intraperitoneal injection of 25mg/kg/10ml, 0.1ml once every day;
      If high dose group: intraperitoneal injection of 75mg/kg/10ml, 0.1ml once every day.
6 Colony counts
   All mice were treated the next day after inoculation, in the fourth week, 6 mice per group were neck broken to death, and 75% alcohol is used for disinfecting the body of each mouse for 5min. The mice were dissected under sterile conditions, spleens and right lungs were separated, then tissues were placed in a mortar, ground, and slowly added to 5ml PBS containing 0.05% Tween-80 for homogenition, 0.1ml homogenate was diluted at a proportion of 1:20, then 0.1ml diluted homogenate was added to 7H9 medium, and allowed to stand for 3 weeks in a 37□ skin box for colony counting.
7 Lung pathological examination
   Left lungs of mice were taken for pathological examination, fixed in 10% formalin solution for dehydration, paraffin embedding, section and other conventional section procedures, and finally HE staining, and histomorphological observation under an optical microscope.
8 Experimental results
   1) Colony count results after treatment for 28 days are as shown in Figure 1, the results show that both the If low dose group and the If high dose group have inhibitory effects on BCG growth of mouse spleens and lungs, the effects of the If high dose group is slightly inferior to those of isoniazide, but obviously superior to those of the model group.
   2) Pathological examination results are as shown in Figure 2, the results show that anti-BCG infection treatment effects of the IF low dose group are obviously superior to those of the solvent group, but inferior to the isoniazide (positive) control group and the If high dose group, and the anti-BCG infection treatment effects of the If high dose group are equivalent to those of the isoniazide (positive) control group.

## Claims

1. Benzothiazinethione derivatives of formula I: 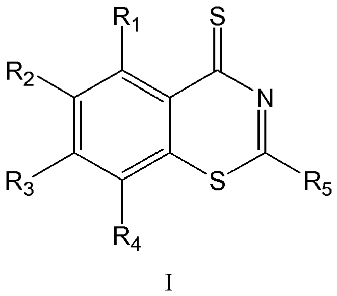 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃;
R₅ is 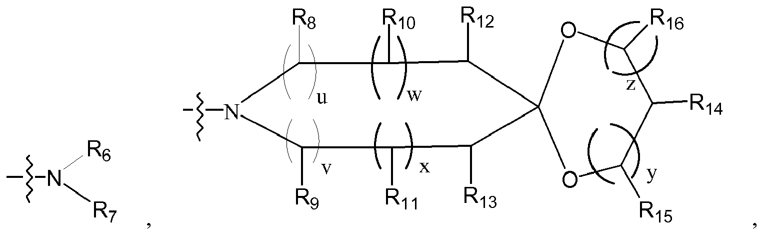 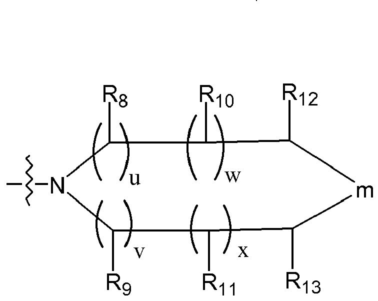 or 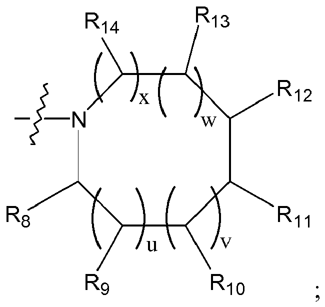 R₆ and R₇ are independently H, C1 - C8 alkyl with substituent, halogen-substituted C1 - C8 alkyl with substituent, phenyl with substituent or pyridyl with substituent; the substituent is H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl;
R₈ - R₁₆ are independently H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, OCF₃, OH, CF₃ or phenyl; and m is N, O or S, u=0-1, v=0-1, w=0-1, x=0-1, y=0-1, z=0-1.

2. The benzothiazinethione derivatives of claim 1, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, NO₂, NH₂, CN or CF₃;
R₅ is 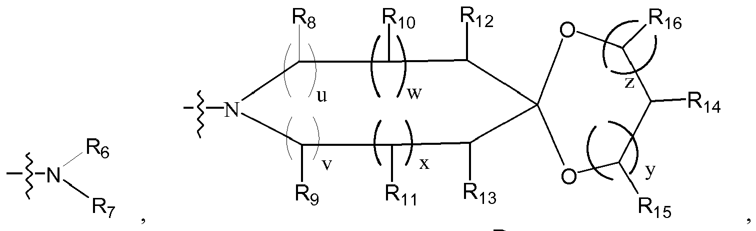 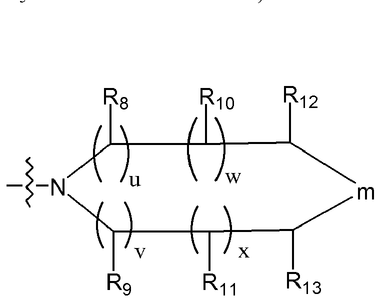 or 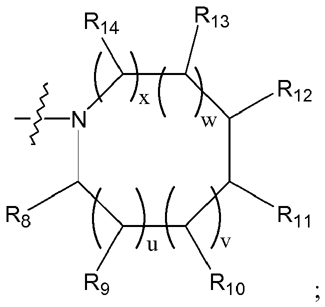
R₆ and R₇ are independently H, C1 - C8 alkyl, halogen-substituted C1 - C8 alkyl, phenyl with substituent, or pyridyl with substituent; the substituent is H, C1 - C8 alkyl, halogen-substituted C1 - C8 alkyl, F, Cl, Br, CF₃, OCF₃, NO₂, NH₂ or CN;
R₈ - R₁₆ are independently H, F, Cl, Br, C1 - C8 alkyl or halogen-substituted C1 - C8 alkyl;
m is N, O or S, u=0-1, v=0-1, w=0-1, x=0-1, y=0-1, z=0-1.

3. The benzothiazinethione derivatives of claim 2, **characterized in that**: R₈ - R₁₆ are independently H, C1 - C8 alkyl or halogen-substituted C1 - C8 alkyl; m is O.

4. The benzothiazinethione derivatives of claim 3, **characterized in that**: R₁ - R₄ are independently H, F, Cl, Br, C1 - C8 alkyl, CF₃ or NO₂; R₆ and R₇ are independently H, C1 - C8 alkyl, phenyl with substituent or pyridyl with substituent, the substituent is H, C1 - C8 alkyl, NO₂, F, Cl, Br or CF₃; R₈ - R₁₆ are H; m is O; u=v=z=0, w=x=y=1.

5. The benzothiazinethione derivatives of claim 4, **characterized in that**: the benzothiazinethione derivatives are:
2-((5-bromopyridine-2-amino)-6,7,8-trifluoro-4H-benzo[e][1,3]thiazine-4-thione,
6,8-dinitro-2-(4-(trifluoromethyl)anilino)-4H-benzo[e][1,3]thiazine-4-thione,
2-((ethylamino)-6,8-dinitro-4H-benzo[e][1,3]thiazine-4-thione,
2-((methylamino)-6,8-dinitro-4H-benzo[e][1,3]thiazine-4-thione,
6,8-dinitro-2-(piperidine-1-yl)-4H-benzo[e][1,3]thiazine-4-thione,
8-nitro-2-(1,4-dioxa-8-azaspiro[4.5]dec-8-yl)-6-(trifluoromethyl)-4H-benzo[e][1,3]th iazine-4-thione,
2-morpholinyl-6,8-dinitro-4H-benzo[e][1,3]thiazine-4-thione, or
2-morpholinyl-8-nitro-6-(trifluoromethyl)-4H-benzo[e][1,3]thiazine-4-thione.

6. The benzothiazinethione derivatives of claim 1, **characterized in that**: R₅ is 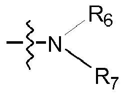 of formula II: 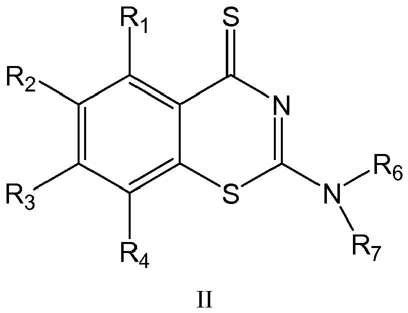 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃;
R₆ and R₇ are independently H, C1 - C8 alkyl with substituent, halogen-substituted C1 - C8 alkyl with substituent, phenyl with substituent or pyridyl with substituent; the substituent is H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl;
or R₆ and R₇ together represent bivalent radicals: 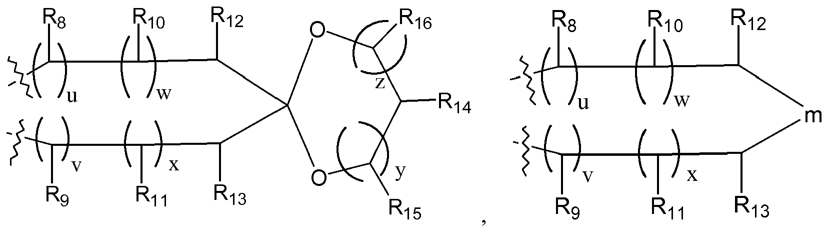 or 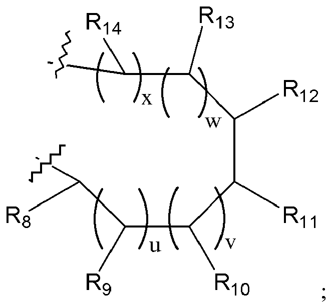 R₈ - R₁₆ are independently H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, OCF₃, OH, CF₃ or phenyl;
m is N, O or S, u=0-1, v=0-1, w=0-1, x=0-1, y=0-1, z=0-1.

7. The benzothiazinethione derivatives of claim 6, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, NO₂, NH₂, CN or CF₃;
R₆ and R₇ are independently H, C1 - C8 alkyl, halogen-substituted C1 - C8 alkyl, phenyl with substituent, or pyridyl with substituent; the substituent is H, C1 - C8 alkyl, halogen-substituted C1 - C8 alkyl, F, Cl, Br, CF₃, OCF₃, NO₂, NH₂ or CN; or R₆ and R₇ together represent bivalent radicals: 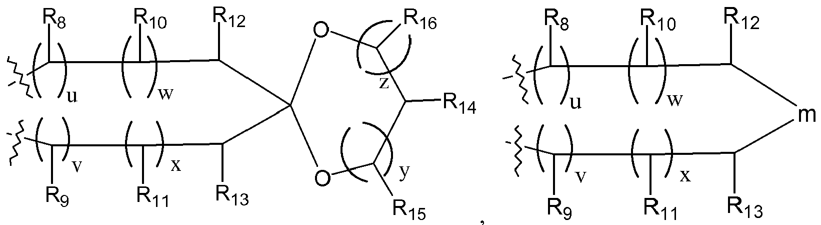 or 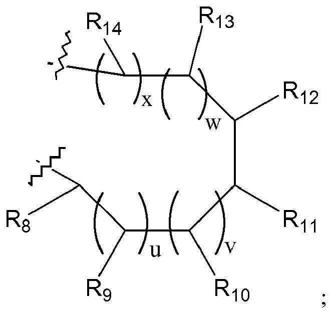
R₈ - R₁₆ are independently H, F, Cl, Br, C1 - C8 alkyl or halogen-substituted C1 - C8 alkyl;
m is N, O or S, u=0-1, v=0-1, w=0-1, x=0-1, y=0-1, z=0-1.

8. The benzothiazinethione derivatives of claim 7, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, NO₂, NH₂, CN or CF₃;
R₆ and R₇ are independently H, C1 - C8 alkyl, halogen-substituted C1 - C8 alkyl, phenyl with substituent, or pyridyl with substituent; the substituent is H, C1 - C8 alkyl, halogen-substituted C1 - C8 alkyl, F, Cl, Br, CF₃, OCF₃, NO₂, NH₂ or CN;
or R₆ and R₇ together represent bivalent radicals: 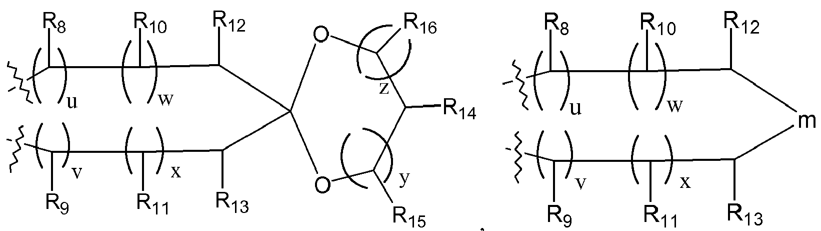 or 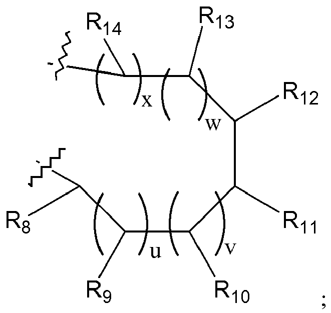
R₈ - R₁₆ are independently H, C1 - C8 alkyl or halogen-substituted C1 - C8 alkyl;
m is O, u=0-1, v=0-1, w=0-1, x=0-1, y=0-1, z=0-1;

9. The benzothiazinethione derivatives of claim 8, **characterized in that**:
R₁ - R₄ are independently H, F, Cl, Br, C1 - C8 alkyl, CF₃ or NO₂;
R₆ and R₇ are independently H, C1 - C8 alkyl, phenyl with substituent or pyridyl with substituent; the substituent is H, C1 - C8 alkyl, NO₂, F, Cl, Br or CF₃;
R₈ - R₁₆ are H; and m is O, u=v=z=0, w=x=y=1.

10. The benzothiazinethione derivatives of claim 1, **characterized in that** R₅ is 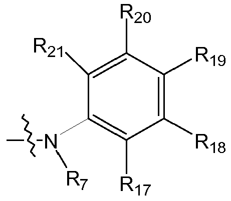 of formula III: 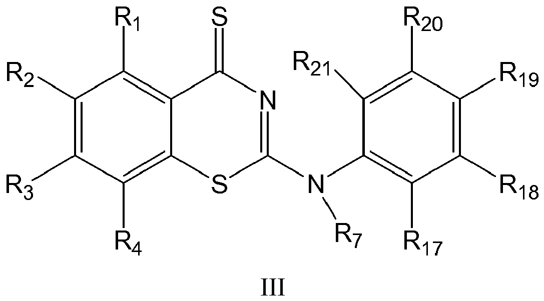 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃;
R₇ is independently H, C1 - C8 alkyl with substituent, halogen-substituted C1 - C8 alkyl with substituent, phenyl with substituent or pyridyl with substituent; the substituent is H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl;
R₁₇ - R₂₁ are independently H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl.

11. The benzothiazinethione derivatives of claim 10, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, OCF₃, NO₂, CN or CF₃; R₇ is independently H, C1 - C8 alkyl, halogen-substituted C1 - C8 alkyl, phenyl with substituent, or pyridyl with substituent; the substituent is H, F, Cl, Br, CF₃, NO₂, C1 - C8 alkyl or halogen-substituted C1 - C8 alkyl; R₁₇ - R₂₁ are independently H, F, Cl, Br, CF₃, NO₂, C1 - C8 alkyl or halogen-substituted C1 - C8 alkyl.

12. The benzothiazinethione derivatives of claim 11, **characterized in that**:
R₁ - R₄ are independently H, F, Cl, Br, C1 - C8 alkyl, halogen-substituted C1 - C8 alkyl, NO₂ or CF₃; R₇ is independently H, C1 - C8 alkyl, halogen-substituted C1 - C8 alkyl, phenyl with substituent, or pyridyl with substituent; the substituent is H, F, Cl, Br, CF₃, NO₂, C1 - C8 alkyl or halogen-substituted C1 - C8 alkyl; R₁₇ - R₂₁ are independently H, F, Cl, Br, CF₃, NO₂, C1 - C8 alkyl or halogen-substituted C1 - C8 alkyl.

13. The benzothiazinethione derivatives of claim 12, **characterized in that**:
R₁ - R₄ are independently H, C1 - C8 alkyl or NO₂; R₇ is independently H or C 1 - C8 alkyl; and R₁₇ - R₂₁ are independently H, CF₃ or C1 - C8 alkyl.

14. The benzothiazinethione derivatives of claim 1, **characterized in that** R₅ is 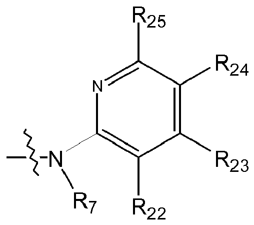 of formula IV: 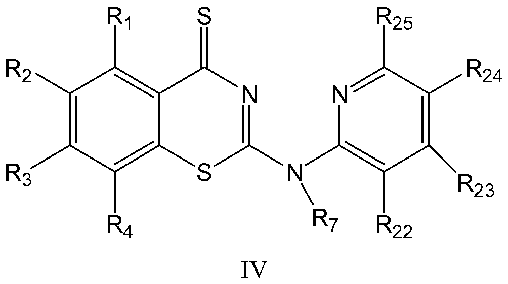 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃;
R₇ is independently H, C1 - C8 alkyl with substituent, halogen-substituted C1 - C8 alkyl with substituent, phenyl with substituent or pyridyl with substituent; the substituent is H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl;
R₂₂ - R₂₅ are independently H, C1 - C8 alkyl, C1 - C8 alkoxyl, C1 - C8 alkyl-substituted sulfamoyl, halogen-substituted C1 - C8 alkyl, C1 - C8 alkyl-substituted carbonyl, C1 - C8 alkyl-substituted aminoacyl, C1 - C8 alkyl-substituted acylamino, halogen, NO₂, OH, OCF₃, CF₃ or phenyl.

15. The benzothiazinethione derivatives of claim 14, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, OCF₃, NO₂, CN or CF₃; R₇ is independent H, C1 - C8 alkyl or halogen-substituted C1 - C8 alkyl; R₂₂ - R₂₅ are independently H, F, Cl, Br, CF₃, NO₂, C1 - C8 alkyl or halogen-substituted C1 -C8 alkyl.

16. The benzothiazinethione derivatives of claim 15, **characterized in that**:
R₁ - R₄ are independently H, F, Cl, Br or C1 - C8 alkyl; R₇ is independently H or C1-C8 alkyl; and R₂₂ - R₂₅ are independently H, F, Cl, Br or C1 - C8 alkyl.

17. The benzothiazinethione derivatives of claim 1, **characterized in that** R₅ is 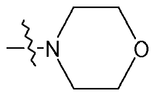 of formula V: 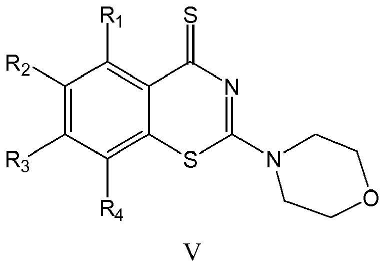 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃.

18. The benzothiazinethione derivatives of claim 17, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, NO₂, OCF₃ or CF₃.

19. The benzothiazinethione derivatives of claim 18, **characterized in that**:
R₁ - R₄ are independently H, C1 - C8 alkyl, CF₃ or NO₂.

20. The benzothiazinethione derivatives of claim 1, **characterized in that** R₅ is 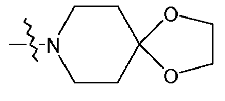 of formula VI: 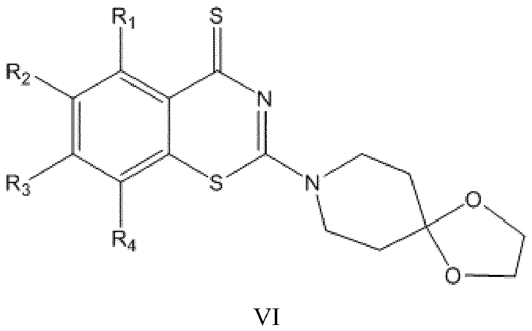 wherein, R₁ - R₄ are independently H, halogen, C1 - C8 alkyl, C1 - C8 alkoxyl, halogen-substituted C1 - C8 alkyl, halogen-substituted C1 - C8 alkoxyl, C1 - C8 alkyl-substituted amino, C1 - C8 alkyl-substituted carbonyl, C1 - C8 alkyl-substituted aminoacyl, C1 - C8 alkyl-substituted acylamino, C1 - C8 alkyl-substituted sulfamoyl, NO₂, NH₂, OCF₃, CN, OH, CHO or CF₃.

21. The benzothiazinethione derivatives of claim 20, **characterized in that**:
R₁ - R₄ are independently H, F, Cl, Br, C1 - C8 alkyl, C1 - C8 alkoxyl, halogen-substituted C1 - C8 alkyl, halogen-substituted C1 - C8 alkoxyl, NO₂, OCF₃ or CF₃.

22. The benzothiazinethione derivatives of claim 21, **characterized in that**:
R₁ - R₄ are independently H, C1 - C8 alkyl, CF₃ or NO₂.

23. A method for synthesizing the benzothiazinethione derivatives of any of claims 1 to 22, **characterized in that**: 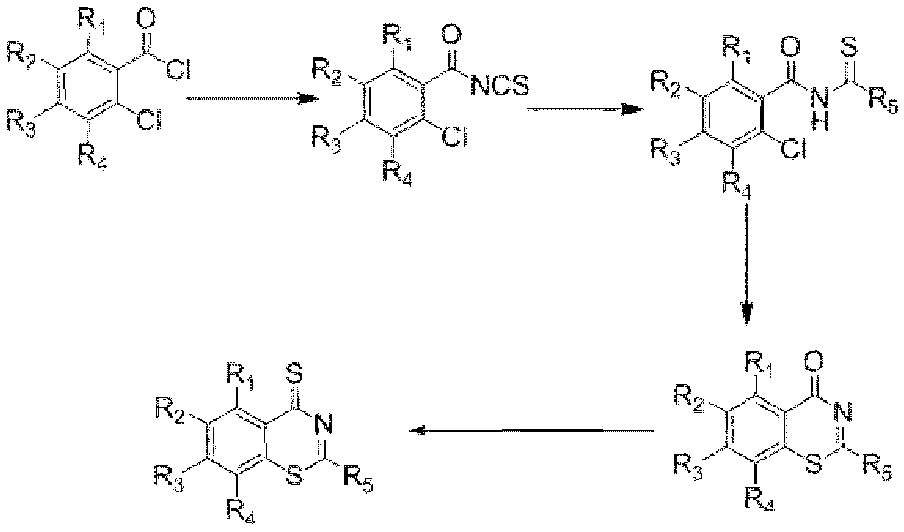 R₁ - R₄-substituted benzoyl chloride reacts with ammonium thiocyanate in the presence of a catalyst to obtain R₁ - R₄-substituted benzoyl isothiocyanate, then the R₁ - R₄-substituted benzoyl isothiocyanate reacts with R₅H by cyclization to obtain R₁ - R₄-substituted benzothiazinone, and finally the R₁ - R₄-substituted benzothiazinone reacts with Lawesson's reagent to obtain R₁ - R₄-substituted benzothiazinethione.

24. The method for synthesizing the compound of claim 23, **characterized in that**: The catalyst is 18-crown-6 or PEG.

25. Uses of the benzothiazinethione derivatives of any of claims 1 to 22 as antitubercular agents.

26. A pharmaceutical composition prepared from the benzothiazinethione derivatives of any of claims 1 to 22 and pharmaceutically acceptable excipients.
